# EUROPEAN PATENT SPECIFICATION

(11) **EP 1 616 533 B1**
(45) Date of publication and mention of the grant of the patent: **27.02.2008**
(21) Application number: 05019033.9
(22) Date of filing: 24.09.2001
(51) Int. Cl.: A61F 2/90

(54) **Intravascular stent apparatus**
Intravaskulärer Stent
Extenseur intravasculaire

(30) Priority: 25.09.2000 US 235180 P
(43) Date of publication of application: 18.01.2006
(62) Divisional of application: 01971318.9
(73) Proprietor: Boston Scientific Scimed, Inc., Maple Grove, MN 55311 (US)
(72) Inventor: Jang, David G., Redlands, CA 92374 (US)
(74) Representative: Hauck Patent- und Rechtsanwälte

(56) References cited:
- EP-A- 0 980 694
- US-A- 6 039 756
- US-A- 6 113 627

## Description

### BACKGROUND OF THE INVENTION

### Field of Invention:

This invention relates to intravascular stents in general, and more particularly to intracoronary stents.

### Description of the Related Art :

Intracoronary stents provide intraluminal scaffolding support of the vascular wall after percutaneous angioplasty in which the balloon catheter is used to expand the stenotic vascular lesion. In both the delivery phase and the deployed phase, there are numerous performance factors that can characterize the overall clinical performance of a stent and can be improved.

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone and without stents, the restenosis rate after angioplasty has been as high as 25-45% in the first time coronary cases. With stents after balloon angioplasty, the restenosis rate has been reduced significantly. Even so, the restenosis rate after stent implantation is reported to be 15-25% range in coronary arteries, depending on the condition of the stented vessel or the specific stent. An ideal coronary stent is still elusive in the current state of the art commercial products.

Some of the best selling current, second generation, stents can be divided into two categories. One category is a stent with high flexibility and the other category has full vessel coverage. The flexible stents generally have poor vessel coverage, tissue prolapse, rough surface modulation and increased restenosis rate. On the other hand, a stent with good vessel coverage in the current state of art may not be flexible enough for easy delivery and for highly efficient procedures. This means that a stent with good flexibility and good vessel coverage remains as the unfulfilled gold standard.

To further reduce the restenosis rate after stent implant, numerous means have been tried including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient, and costly. Brachytherapy is a radioactive device and a radiation therapy specialist from another department has to be involved with the interventional cardiologist in the cardiac catheterization laboratory. The laser and atherectomy devices proved to be marginally useful with added costs.

Local drug therapy appears to be a very promising method for the future, as better pharmaceutical, chemical, or biogenetic agents are developed and became available. Some research data, both from animal tests and human clinical studies indicate evidence of some suppression of restenosis after stent implantation when certain growth blocking pharmaceutical agents coat the stent.

In other instances, it has been speculated that certain surface modifying materials coated on the surface of the stent may be beneficial, alone or in combination with growth suppressing agents, in reducing the restenosis rate. In either instance, a drug or substance should be locally attached or coated on the stent in sufficient amounts. However, attaching or coating a sufficient amount of a substance or drug on the coronary stent may not be an easy proposition, because coating enough volume of the drug on the small surface area of a stent is a challenging task. If and when stent coating becomes practical, a good stent can still have better outcomes than a poorly designed stent when used with substance coating.

A stent is a scaffolding device. When delivered to a remote vessel location via percutaneous approach it can be deployed by expanding the device inside a vessel. The vessel can have a very small caliber and sometimes has a very tortuous anatomy. When a stent is deployed, the stent should have a good radial strength, good vessel coverage, a good internal surface modulation without tulips (i. e., sharp metal loop projections that resemble fish scale phenomena), optimal vesselconformability, a low metal fraction, and so forth.

If the stent is stiff and non-flexible, it can be very difficult to deliver to an intended lesion site inside a vessel. Easy delivery of a stent is aided by good flexibility of the stent in combination with the delivery balloon, a smooth surface modulation without or minimizing tulips and a degree of radiopacity. A good stent should have a combination of features for delivery and deployment.

Although there are countless variations of vascular stent designs today, few have these desired stent features both in the delivery phase and in the postdelivery phase. Today's top selling stents in the market can have undesirable characteristics, either in the delivery phase or in the deployed phase of the stent life cycle. For example, some stents may have flexibility, but lack vessel coverage or surface modulations both in delivery and deployed phases. Some stents may have good vessel coverage and surface modulations, but lack flexibility.

Vascular stents, which are designed to be delivered to vessel sites via percutaneous approach, can have two elements. The first element is the expansion strut that expands circumferentially to provide the scaffolding radial force against a possible collapsing force of the vessel wall. The second element is the connecting strut that can link the expansion struts along the longitudinal axis of the stent, giving articulation or flexibility to the stent. The particular combination of expansion struts and connecting struts generally form various cells, depending on the specific configuration and shape of the expansion and connecting struts. If a cell is too large, the vessel wall support or coverage can be poor and the vessel wall tissue can prolapse through the large cells of the stent net. If the cells are too small, the vessel wall may be well covered but the metal fraction of the stent can be too high. The metal fraction is a fraction of the total metal surface area of an expanded stent (inside a blood vessel) divided by the total internal vessel wall surface area where the stent is deployed.

Some very flexible stents have very large cell size with poor vessel coverage and tissue prolapse, in addition to poor(inner and/or outer) surface modulation due to large numbers of tulips directed to both ends of the stent.

Most of the current flexible stents are designed to effect flexibility by using fewer or a minimal number of connecting struts, handicapping the vessel coverage, surface modulation and tissue prolapse defects.

On the other hand, a stent that is designed for good vessel coverage and ideal cell size tends to be inflexible when such a stent is being delivered to a vessel lesion. A lack of flexibility during stent delivery is a very critical issue; a stiff stent often cannot be delivered to a needed location inside a blood vessel because such a stent cannot navigate through a tortuous and small vessel lumen.

US 6,113,627 discloses a stent including a plurality of expansion struts forming a first expansion column, the first expansion column includes a first expansion strut, a second expansion strut and a first joining strut. The first joining strut couples a distal end of the first expansion strut to a distal end of the second expansion strut, and the first expansion strut has a stepped distal portion and the second expansion strut has a stepped proximal portion. A plurality of expansion struts defines a second expansion column, and the second expansion column includes a first expansion strut, a second expansion strut and first joining strut which couples a distal end of the first expansion strut to a distal end of the second expansion strut, and the first expansion strut has a stepped proximal portion and the second expansion strut has a stepped distal portion. A first serial connecting strut column is formed of a plurality of serial connecting struts and includes a first serial connecting strut. The first serial connecting strut column couples the first expansion strut column to the second expansion column. The serial connecting strut column comprises connecting struts which include an angle with the main stent direction.

US 6,039,756 discloses a stent having a plurality of first column expansion strut pairs that form a first expansion column. Furthermore, a plurality of second column expansion strut pairs forms a second expansion strut column and a plurality of first serial connecting struts forms a first connecting strut column. Different geometries for the connecting struts are described. Figure 6 shows a stair-stepped geometry for the connecting strut with the intermediate section of the connecting strut extending along the main direction of the stent.

The technical problem of the invention is to provide a vascular stent that is very flexible for delivery and with good vessel coverage when deployed.

The problem is solved according to claim 1.

The stent according to the invention includes a combination of maximum possible flexibility and conformability in the stent, full vessel coverage with optimal metal fraction, evenly expanding stent struts, excellent radial strength and radiopacity, and smooth surface modulations in both delivery and deployed phases of the stent life cycle.

The stent according to the invention includes a first expansion column, a second expansion column, and a first connecting strut column. The first expansion column and the second expansion column can each include individual expansion struts forming a plurality of expansion strut pairs. Two adjacent expansion strut pairs can share a common strut. The first connecting strut column can include a plurality of individual first connecting struts that couple the first and second expansion columns. Each connecting strut includes a curvilinear proximal section and a curvilinear distal section.

### BRIEF DESCRIPTION OF DRAWINGS

Figure 1 shows a side elevation view of an embodiment of a stent, such as a tubular stent.
Figure 2 shows an isometric view of an embodiment of a stent, such as a tubular stent.
Figure 3 shows a cut-open view of an embodiment of a stent. Various expansion columns and connecting strut columns are shown.
Figure 4 shows a magnified view of a middle section of an embodiment of a stent, such as a stent of Figures 1, 2, and 3. Some details are shown of expansion columns.
Figure 5 shows a magnified view of a middle section of an embodiment of a stent, such as a stent of Figures 1, 2, and 3. Some details are shown of connecting strut columns.

### DETAILED DESCRIPTION OF DRAWINGS

Some embodiments of stents can be in a state, such as one or more of a non-expanded state, an expanded state, a crimped state, and a non-crimped state.
Some embodiments of stents can include one or more of a first expansion column, a second expansion column, a third expansion column, a first connecting strut column, and a second connecting strut column.

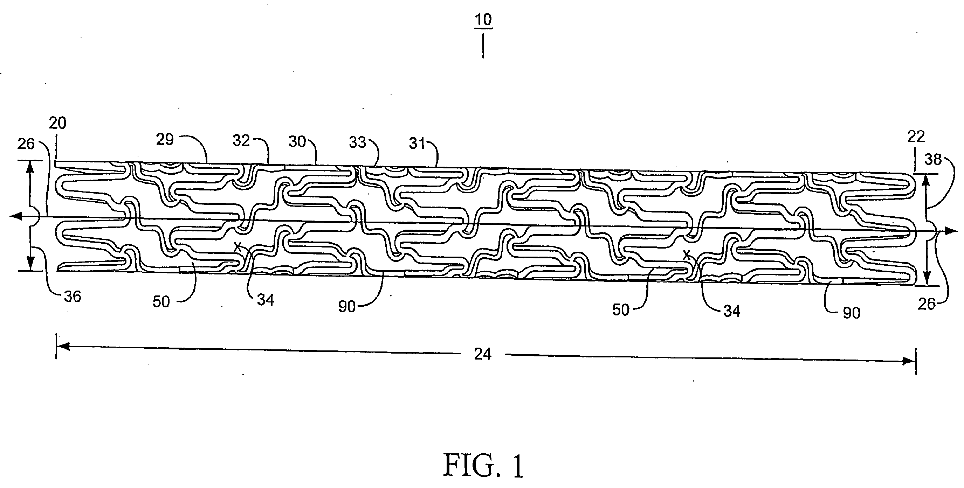

Figure 1 shows an embodiment having a first expansion column 29, a second expansion column 30, a third expansion column 31, a first connecting strut column 32, and a second connecting strut column 33. The first expansion column, the second expansion column, and the third expansion column can include individual expansion struts forming a plurality of expansion strut pairs.

In many embodiments of the stent, two adjacent expansion strut pairs share a common strut.

The first connecting strut column and the second connecting strut column include a plurality of individual connecting struts. Each connecting strut has a stair-step geometric configuration with a curvilinear proximal section and a curvilinear distal section. The first connecting strut column can include individual first connecting struts and the second connecting strut column can include individual second connecting struts. The first connecting column couples the first and second expansion columns. Figure 1 shows an example where the first connecting column 32 couples the first expansion column 29 and the second expansion column 30. The second connecting column 33 couples the second expansion column 30 and the third expansion column 31.

Each expansion strut can have a stair-step configuration. Distal ends of expansion strut pairs of the first expansion column that are coupled to proximal ends of expansion strut pairs of the second expansion column can be vertically offset. Distal ends of expansion strut pairs of the second expansion column that are coupled to proximal ends of expansion strut pairs of the third expansion column can also be vertically offset.

Some embodiments of the stent include connecting struts with five sections, including an intermediate section, a proximal curvilinear section and a distal curvilinear section. The proximal section of each first connecting strut can be contralaterally conjoined to an expansion strut pair of the first expansion column. The distal section can be contralaterally conjoined to an expansion strut pair of the second expansion column. The proximal and distal sections can have the same lengths.

At least a portion of the proximal and distal curvilinear section can be parallel to a portion of an expansion strut pair loop in the first expansion column or in the second expansion column.

The proximal section can include a terminal end conjoined to an expansion strut in the first expansion column, and at least one surface that is conjoined to at least one surface of an expansion strut in the first expansion column. The distal section includes a terminal end conjoined to an expansion strut in the second expansion column, and at least one surface that is conjoined to an expansion strut in the second expansion column.

At least one of the proximal and distal sections of each connecting strut can be contralaterally conjoined to an expansion strut pair of the first and second expansion columns, or to an expansion pair of the second and third expansion columns.

At least a portion of the curvilinear proximal section of each connecting strut can be parallel to a portion of an expansion strut pair loop. At least a portion of the curvilinear distal section of each connecting strut can be parallel to a portion of an expansion strut pair loop of an expansion column.

At least a portion of the curvilinear proximal section of each connecting strut can be positioned in close proximity to an expansion strut pair loop of an expansion column. At least a portion of the curvilinear distal section of each connecting strut can be positioned in close proximity to an expansion strut pair loop. Close proximity can be in the range of 0.001 to 0.050 (0.025 mm - 1.3 mm) of an inch, in the range of 0.001 to 0.040 (0.025 mm - 1.02 mm) of an inch, or in the range of 0.001 to 0.030 (0.025 mm - 0.76 mm) of an inch.

In various embodiments of the stent each connecting strut can have a proximal end, a distal end, four pivot points, and a longitudinal axis. The proximal end can extend in a first direction. The distal end of a connecting strut can extend in an second direction opposite to the first direction.

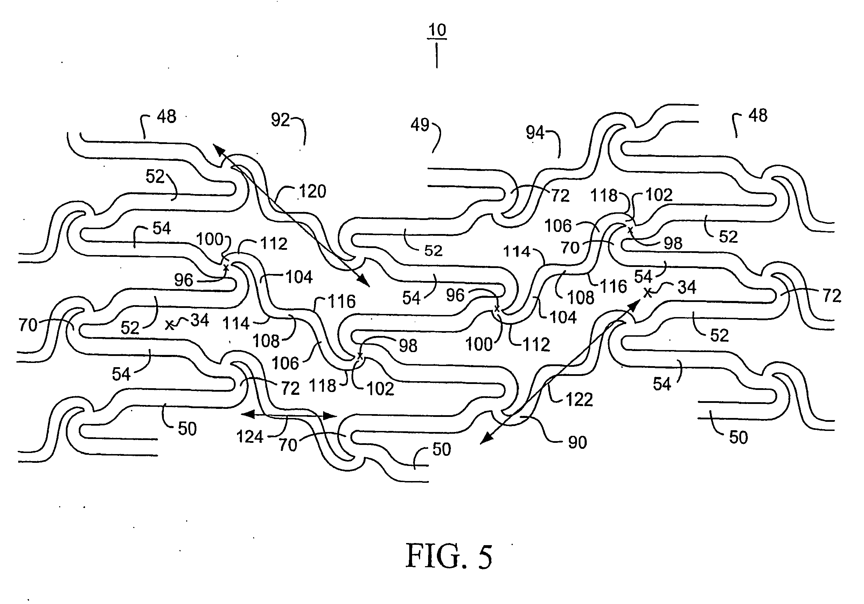

Each connecting strut in the first connecting strut column has two radii of curvature each with the proximal and distal curvilinear section. Figure 5 shows examples pivot points 112 and 114 having radii of curvature in the proximal curvilinear section, and pivot points 116 and 118 having radii of curvatures in the distal curvilinear section. Each pivot point can have at least one radius of curvature.

The longitudinal axis of the connecting strut may be non-parallel to a longitudinal axis of the stent. In some embodiments of the stent, each connecting strut in a same connecting strut column can share the similar longitudinal axis, which can be mutually parallel.

Each first connecting strut can have a longitudinal axis that extends in a first direction. Each second connecting strut can have a longitudinal axis that extends in an opposite second direction.

The intermediate section can have a longitudinal axis. The longitudinal axis of an intermediate section may be parallel to an expansion strut in the first expansion column, parallel to an expansion strut in the second expansion column, non-parallel to the longitudinal axis of the first connecting strut, and parallel to the longitudinal axis of the stent. The intermediate section is coupled to the curvilinear proximal section and the curvilinear distal section.

Each connecting strut is contralaterally conjoined to the first and second expansion columns. At least a portion of the connecting struts can have asymmetrical geometric configurations.

Some embodiments of the stent include a first end expansion column and a second end expansion column. The first and second end expansion columns can define a proximal and a distal end of the stent, and are mirror images of each other.

A plurality of cells can be defined by the first expansion column, the second expansion column and the first connecting strut column. Cells can have evenly spaced, asymmetrical geometric shapes. Cells can also have evenly spaced geometric shapes with a quasi-hexagonal geometry in a nominally expanded state.

Expansion strut pair loops in two adjacent expansion columns can be aligned in a peak-to-valley, in a valley-to-peak geometry, or in a peak-to-peak geometry.

Various embodiments of the stent include one or more types of expansion columns. A first expansion column includes various expansion strut pairs. A joining strut section at a proximal end can join an expansion strut with a short stepped-down section at a proximal end and an expansion strut with a short stepped-down section at a distal end, forming an expansion strut pair loop.

A joining strut section at a distal end can join an expansion strut with a short stepped-down section at a distal end and an expansion strut with a short stepped-down section at a proximal end, forming an expansion strut pair loop.

These expansion strut pairs alternate, for example for six cycles, around the expansion ring without a break.

A second expansion column includes various expansion strut pairs. A joining strut section at a proximal end can join an expansion strut with a short stepped-up section at a proximal end and an expansion strut with a short stepped-up section at a distal end, forming an expansion strut pair loop. A joining strut section at a distal end can join an expansion strut with a short stepped-up section at a distal end and an expansion strut with a short stepped-up section at a proximal end, forming an expansion strut pair loop. These expansion strut pairs alternate, for example for six cycles, around the expansion ring without a break.

Different types of expansion columns can be arranged in an alternating sequence, inter-linked along the length of the stent by connecting columns.

Stepped-up or stepped-down segments with a sloped transitional section can provide flexibility, smooth surface modulation effects, and well-formed crimping space to the stent. A connecting strut can conjoin with an expansion strut pair at a short stepped-down or stepped-up section of an expansion strut. A connecting strut can be a direct extension of an expansion strut and be integral to the stent structure rather than a separate structure added, welded, or attached.

Separate terminology for stent elements, for example expansion and connecting struts, conveniently describing the anatomy and function of various stent portions.

Connecting struts can have a curvilinear double stair step shape with a longitudinal axis diagonally tilted to one side or the other side from the vertical, due to the diagonal orientation. Connecting struts of different connecting strut columns can have different longitudinal axes, which can be mirror images. In some embodiments of the stent, a connecting strut has three segments, two endstem sections and four pivot points. Pivot points can have a varying radius of curvature. These multiple pivot points are responsible for flexibility. One end of a connecting strut can conjoin with an expansion strut pair in one expansion strut column and another end of the connecting strut can conjoin to another expansion strut pair in an adjacent expansion strut column. The connecting strut can link two apposing expansion strut pairs in a diagonal orientation. A diagonal orientation of a connecting strut of the stent gives added flexibility, excellent crimping, vessel conformability and smooth surface modulation to the stent.

Further, when a connecting strut conjoins expansion strut pairs, both ends of a connecting strut conjoin to the contralateral sides of apposing expansion strut pairs of adjacent expansion columns, at a stepped down or a stepped up sections. Conjoining a connecting strut on contralateral sides, along with a diagonal orientation and multiple pivot points, can provide good stent performance characteristics.

In some embodiments of the stent, the ratio of expansion strut to connecting strut number is two to one, where such as when a connecting strut is conjoined to expansion strut pairs.

When the expansion columns and connecting columns are conjoined, the stent can have a continuous, unbroken cylindrical form without breaks or delinking around the circumference and along the length of the stent. Unbroken links between the expansion and connecting struts can make regular and evenly spaced asymmetrical cells. The cell size can be maximized or minimized by programming of the stent design platform, as dictated by clinical or applications requirements.

Figure 1 shows one embodiment of a stent 10 in side elevation view, with a first expansion column 29, a second expansion column 30, a third expansion column 31, a first connecting strut column 32, and a second connecting strut column 33. The stent 10 has a proximal end 20 and a distal end 22. The stent 10 has a tubular or cylindrical structure. The stent 10 has a longitudinal length 24 and a longitudinal axis 26.

In some embodiments of the stent, an expansion column can be a zigzag or corrugated ring configuration of expansion struts. An expansion column, for example expansion column 30, in a stent 10 can be an unbroken circular ring.

Multiple expansion strut columns can be interconnected with connecting struts continuously along the longitudinal axis 26 of the stent 10 in an unbroken manner to form a stent 10 having a tubular shape. The interconnections among expansion columns and connecting strut columns enclose spaces, or cells, formed by expansion struts and connecting struts. In the embodiment shown in Figure 1, all cells have asymmetrical geometry. The stent 10 has two different diameters, including an outer diameter 36 and an inner diameter 38, having a difference of a thickness of the stent 10. Both the outer diameter 36 and inner diameter 38 can change as the stent 10 goes through a crimping stage, when the diameters 36 and 38 are narrowed, and through a deployed stage, when the diameters 36 and 38 are expanded.

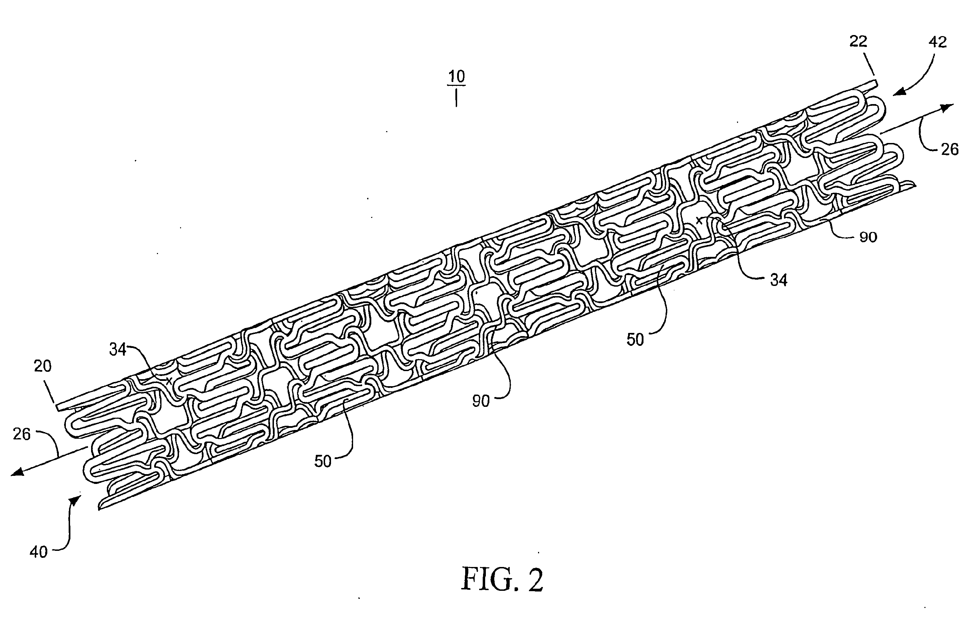

Figure 2 shows one embodiment of a stent 10 in isometric view. A back half of the stent 10 can be seen through the cell space of the front half of the stent 10. The shown embodiment of the stent 10 has a tubular structure with a central lumen, a proximal opening 40, and a distal opening 42. Stent cells 34 include open spaces in the network of expansion struts and connecting struts.

The lumen includes the central, open tunnel formed by the expansion and connecting struts of the stent.

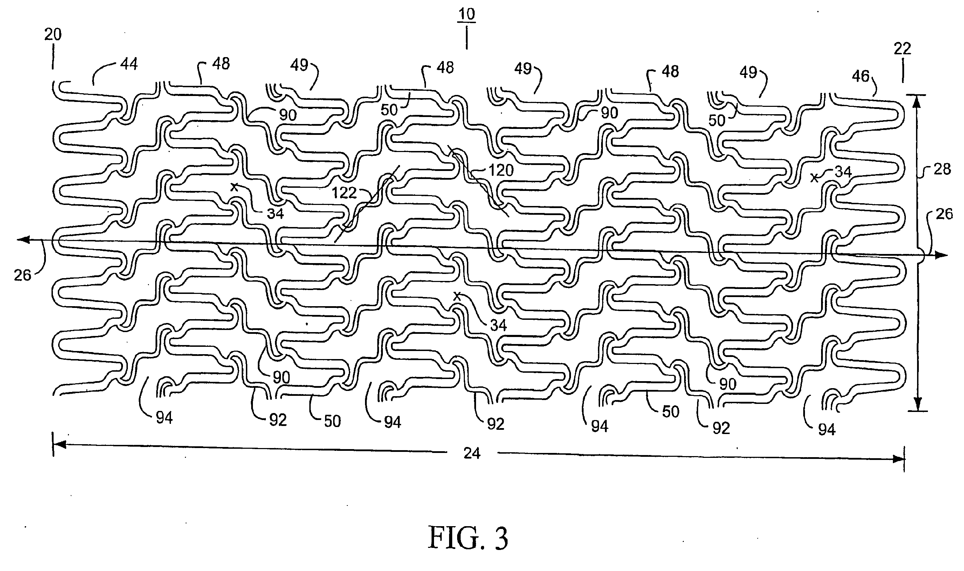

Figure 3 shows one embodiment of a stent 10 in cut-open 2-dimensional view. The stent 10 has a proximal end 20 and a distal end 22. This view of the stent 10 is a scale drawing for a 15 mm coronary stent. There are eight expansion columns and seven connecting strut columns. At the proximal end 20 is an expansion column 44, which is a mirror image of an expansion columns 46 at the distal end 22. In the middle of the stent 10, there are six expansion columns, such that an expansion column 49 alternates with an expansion column 48. Interconnecting with eight expansion columns along the longitudinal axis 26 of the stent 10 are seven connecting strut columns including four connecting strut columns 94 and three connecting strut columns 92, such that a connecting strut column 94 alternates with a connecting strut column 92.

There are a total of 42 cells of various asymmetric configurations. All the cells in this embodiment have asymmetrical geometry. Expansion columns 44,46, 48, and 49 are vertically arranged with expansion strut pair loops aligned peak to-valley. Connecting strut columns 92 and 94 interconnect expansion columns 44,46,48, and 49 in a continuous and unbroken manner along the length 24 and around the circumference 28 of the stent 10.

The stent 10 in Figure 3 has the proximal end 20 on the left and the distal end 22 on the right. The stent 10 has a length 24 horizontally and a circumference 28 vertically, with a longitudinal axis 26 horizontally along the length 24 from the proximal end 20 to the distal end 22.

A width (horizontal dimension) of expansion columns is wider than a width of connecting strut columns. However, a width of a connecting strut column could be made the same or larger than a width of an expansion column.

The variation of width ratio between a connecting strut column and an expansion column are within the scope of present invention of stent 10. The number of expansion strut cycles in an expansion column and the number of connecting struts in a connecting strut column can be made variably different.

Variable numbers of making expansion strut cycles and connecting struts are within the scope of the present invention of the stent 10.

In some embodiments of the stent, one type of expansion column includes various expansion strut pairs. A joining strut section at a proximal end conjoins an expansion strut with short stepped-down section at a proximal end and an expansion strut with a short stepped-down section at a distal end, forming an expansion strut pair loop. A joining strut section at a distal end conjoins an expansion strut with a short stepped-down section at a distal end and an expansion strut with a short stepped-down section at a proximal end, forming an expansion strut pair loop. These expansion strut pairs alternate, for example for six cycles, around the expansion ring without a break.

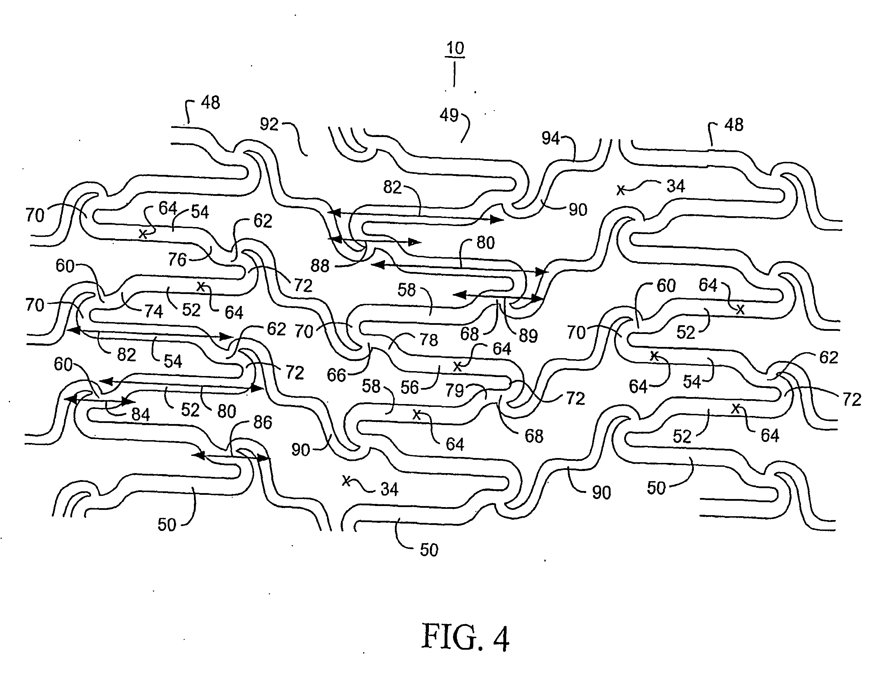

Figure 4 shows an embodiment having this type of expansion column 48. A joining strut section 70 at a proximal end conjoins an expansion strut 52 with a short stepped-down section at a proximal end and an expansion strut 54 with a short stepped-down section at a distal end forming an expansion strut pair loop. A joining strut section 72 at a distal end conjoins an expansion strut 54 with a short stepped-down section at a distal end 62 and an expansion strut 52 with a short stepped-down section at a proximal end 60, forming an expansion strut pair loop.

Another type of expansion column includes various expansion strut pair combinations. A joining strut section at a proximal end conjoins an expansion strut with a short stepped-up section at a proximal end and an expansion strut with a short stepped-up section at a distal end, forming an expansion strut pair loop. A joining strut section at a distal end conjoins an expansion strut with a short stepped-up section at a distal end and an expansion strut with a short stepped-up section at a proximal end, forming an expansion strut pair loop.

Figure 4 shows an embodiment having this type of expansion column 49. A joining strut section 70 at a proximal end conjoins an expansion strut 56 with a short stepped-up section at a proximal end 66 and an expansion strut 58 with a short stepped-up section at a distal end 68, forming an expansion strut pair loop. A joining strut section 72 at a distal end can join an expansion strut 58 with a short stepped-up section at a distal end 68 and an expansion strut 56 with a short stepped-up section at a proximal end 66, forming an expansion strut pair loop.

These proximal and distal expansion strut pairs alternate, for example for six cycles, around the expansion ring without a break.

A transitional slope 74 can be between a stepped down proximal section 60 and a straight section 64 in a stair step expansion strut 52. Likewise, a transitional slope 76 can be between a stepped down distal section 62 and a straight section 64 in a stair step expansion strut 54. A transitional slope 78 can be between a stepped up proximal section 66 and a straight section 64 in a stair step expansion strut 56. Likewise, a transitional slope 79 can be between a stepped up distal section 68 and a straight section 64 in a stair step expansion strut58.

Figure 5 shows an example of connecting strut column 92 and connecting strut column 94. Connecting struts can have a curvilinear double stair step shape with a longitudinal axis is tilted to one side or the other side from the vertical plane, due to the diagonal orientation of connecting struts.

Connecting struts of different connecting strut columns can have different longitudinal axes, which can be mirror images. For example, longitudinal axis 120 for connecting struts in connecting strut column 92 is different from longitudinal axis 122 for connecting struts in connecting strut column 94. In some embodiments of the stent, a connecting strut has three segments, two endstem sections and four pivot points. Figure 5 shows connecting struts with a proximal curvilinear segment 104, central segment 108, distal curvilinear segment 106, proximal end-stem 100, distal end-stem 102, and pivot points 112, 114,116, and 118. Pivot point 112 is a junction between proximal end-stem 100 and proximal curvilinear segment 104, pivot point 114 is a junction between proximal curvilinear segment 104 and central segment 108, pivot point 116 is a junction between central intermediate segment 108 and distal curvilinear segment 106, and pivot point 118 is a junction between distal curvilinear segment 106 and distal end-stem 102. These pivot points can have a varying degree of radius of curvature. These multiple pivot points are responsible for flexibility of the stent and for prevention of foreshortening of the stent. One end of a connecting strut can conjoin with an expansion strut pair in one expansion strut column and another end of the connecting strut can conjoin to another expansion strut pair in an adjacent expansion strut column. For example, a connecting strut in connecting strut column 92 has a proximal end 96 conjoined to an expansion strut in one expansion column, and a distal end 98 conjoined to an expansion strut in another expansion column. Proximal end 96 and distal end 98 of the connecting strut are conjoined to contralateral sides of apposing expansion strut pairs of adjacent expansion columns, at a stepped down or a stepped up sections. Conjoining a connecting strut on contralateral sides, along with a diagonal orientation and multiple pivot points of the connecting strut provides good stent performance characteristics. The connecting strut can link two apposing expansion strut pairs in a diagonal orientation. A diagonal orientation of a connecting strut of the stent gives added flexibility, excellent crimping, vessel conformability and smooth surface modulation to the stent.

## Claims

1. A stent comprising:
• a first expansion column (48) including individual expansion struts forming a plurality of expansion strut pairs (52, 54) in a ring shape, wherein two adjacent expansion strut pairs share a common strut, wherein one expansion strut (52) of an expansion strut pair of the first expansion column (48) has a stair-step segment at a proximal section and the other expansion strut (54) of the expansion strut pair has a stair-step segment at a distal section;
• a second expansion column (49) including individual expansion struts (52, 54) forming a plurality of expansion strut pairs (52, 54) in a ring shape, wherein two adjacent expansion strut pairs share a common strut, wherein one expansion strut (54) of an expansion strut pair of the second expansion column (49) has a stair-step segment at a proximal section and the other expansion strut (52) of the expansion strut pair has a stair-step segment at a distal section, a first connecting strut column (92) including a plurality of individual first connecting struts that couple the first and second expansion columns, wherein each of a first connecting strut in the first connecting strut column has a stair-step geometric configuration,
**characterized in that** each first connecting strut in the first connecting strut column has an intermediate section (108) coupled to a curvilinear proximal section (104) and a curvilinear distal section (106), wherein the proximal and distal sections (104, 106) of each first connecting strut of the first connecting strut column have the same lengths, wherein the intermediate section (108) has a longitudinal axis (124) extending in a longitudinal direction of the stent, and wherein the proximal section (104) of each first connecting strut in the first connecting strut column (92) is contralaterally conjoined to an expansion strut pair of the first expansion column (48) at a stepped down or stepped up section and its corresponding distal section (108) is contralaterally conjoined to an expansion strut pair of the second expansion column (49) at a stepped down or stepped up section.

2. The stent of claim 1, wherein each first connecting strut in he first connecting strut column (92) has a proximal end that extends in a first direction and a distal end that extends in an opposite second direction.

3. The stent of claim 1, wherein each first connecting strut in the first connecting strut column has four pivot points (112, 114, 116, 118).

4. The stent of claim 3, wherein each pivot point (112, 114, 116, 118) has at least one radius of curvature.

5. The stent of claim 1, wherein each first connecting strut in the first connecting strut column (92) has a longitudinal axis (112) that is non-parallel to a longitudinal axis of the stent.

6. The stent of claim 1, wherein each intermediate section (108) of each first connecting strut of the first connecting strut column (92) has a longitudinal axis (124) that is non-parallel to the longitudinal axis (120, 122) of the first connecting strut.

7. The stent of claim 6, wherein each first connecting strut in the first connecting strut column has the same longitudinal axis in a cut-open 2-dimensional view.

8. The stent of claim 6, wherein all of the first connecting struts in the first connecting strut column have parallel longitudinal axes in a cut-open 2-dimensional view.

9. The stent of claim 1, wherein at least a portion of the first connecting struts of the first connecting strut column have asymmetrical geometric configurations.

10. The stent of claim 1, wherein a terminal end of the proximal section (104) of each first connecting strut in the first connecting column (92) is conjoined to an expansion strut in the first expansion column, and a terminal end of the distal section (106) of each first connecting strut is conjoined to an expansion strut in the second expansion column.

11. The stent of claim 1, wherein the proximal section (104) of each first connecting strut has a surface that is conjoined to at least one surface of an expansion strut in the first expansion column, and the distal section (106) of each first connecting strut has at least one surface that is conjoined to an expansion strut in the second expansion column.

12. The stent of claim 1, wherein each first connecting strut of the first connecting column (92) is contralaterally conjoined to the first and second expansion columns (48, 49).

13. The stent of claim 1, wherein at least a portion of the curvilinear proximal section (104) of each first connecting strut of the first connecting strut column (92) is in close proximity to an expansion strut pair of the first expansion column (48).

14. The stent of claim 1, wherein at least a portion of the curvilinear proximal section (104) of each first connecting strut of the first connecting strut column (52) is parallel to a portion of an expansion strut pair of the first expansion column (48).

15. The stent of claim 14, wherein at least a portion of the curvilinear distal section (106) of each first connecting strut of the first connecting strut column (92) is in close proximity to an expansion strut pair of the second expansion column (49).

16. The stent of claim 1, wherein at least a portion of the curvilinear distal section (106) of each first connecting strut of the first connecting strut column (92) is parallel to a portion of an expansion strut pair of the second expansion column (49).

## Patentansprüche

1. Stent, umfassend:
• eine erste Ausdehnungssäule (48), umfassend einzelne Ausdehnungsstreben, die eine Mehrzahl Ausdehnungsstrebenpaare (52, 54) in einer Ringform bilden, wobei zwei benachbarte Ausdehnungsstrebenpaare eine gemeinsame Strebe teilen, wobei eine Ausdehnungsstrebe (52) eines Ausdehnungsstrebenpaars der ersten Ausdehnungssäule (48) ein Stufensegment an einem proximalen Abschnitt aufweist und die andere Ausdehnungsstrebe (54) des Ausdehnungsstrebenpaars ein Stufensegment an einem distalen Abschnitt aufweist;
• Eine zweite Ausdehnungssäule (49), umfassend einzelne Ausdehnungsstreben (52, 54), die eine Mehrzahl Ausdehnungsstrebenpaare (52, 54) in einer Ringform bilden, wobei zwei benachbarte Ausdehnungsstrebenpaare eine gemeinsame Strebe teilen, wobei eine Ausdehnungsstrebe (54) eines Ausdehnungsstrebenpaars der zweiten Ausdehnungssäule (49) ein Stufensegment an einem proximalen Abschnitt aufweist und die andere Ausdehnungsstrebe (52) des Ausdehnungsstrebenpaars ein Stufensegment an einem distalen Abschnitt aufweist, eine erste Verbindungsstrebensäule (92), umfassend eine Mehrzahl einzelner erster Verbindungsstreben, die die erste und zweite Ausdehnungssäule koppeln, wobei jede der ersten Verbindungsstreben in der ersten Verbindungsstrebensäule eine geometrische Stufenkonfiguration aufweist,
**dadurch gekennzeichnet, dass** jede erste Verbindungsstrebe in der ersten Verbindungsstrebensäule einen Zwischenabschnitt (108) aufweist, der an einen krummlinigen proximalen Abschnitt (104) und einen krummlinigen distalen Abschnitt (106) gekoppelt ist, wobei der proximale und der distale Abschnitt (104, 106) jeder ersten Verbindungsstrebe der ersten Verbindungsstrebensäule die gleiche Länge aufweisen, wobei der Zwischenabschnitt (108) eine Längsachse (124) aufweist, die sich in einer Längsrichtung des Stents erstreckt, und wobei der proximale Abschnitt (104) jeder ersten Verbindungsstrebe in der ersten Verbindungsstrebensäule (92) kontralateral mit einem Ausdehnungsstrebenpaar der ersten Ausdehnungssäule (48) bei einem Stufenabschnitt nach unten oder einem Stufenabschnitt nach oben verbunden ist und sein entsprechender distaler Abschnitt (108) kontralateral mit einem Ausdehnungsstrebenpaar der zweiten Ausdehnungssäule (49) bei einem Stufenabschnitt nach unten oder einem Stufenabschnitt nach oben verbunden ist.

2. Stent nach Anspruch 1, wobei jede erste Verbindungsstrebe in der ersten Verbindungsstrebensäule (92) ein proximales Ende aufweist, das sich in einer ersten Richtung erstreckt, und ein distales Ende, das sich in einer entgegengesetzten zweiten Richtung erstreckt.

3. Stent nach Anspruch 1, wobei jede erste Verbindungsstrebe in der ersten Verbindungsstrebensäule vier Drehpunkte (112, 114, 116, 118) aufweist.

4. Stent nach Anspruch 3, wobei jeder erste Drehpunkt (112, 114, 116, 118) mindestens einen Krümmungsradius aufweist.

5. Stent nach Anspruch 1, wobei jede erste Verbindungsstrebe in der ersten Verbindungsstrebensäule (92) eine Längsachse (112) aufweist, die nicht parallel zu einer Längsachse des Stents ist.

6. Stent nach Anspruch 1, wobei jeder Zwischenabschnitt (108) jeder ersten Verbindungsstrebe der ersten Verbindungsstrebensäule (92) eine Längsachse (124) aufweist, die nicht parallel zur Längsachse (120, 122) der ersten Verbindungsstrebe ist.

7. Stent nach Anspruch 6, wobei jede Verbindungsstrebe in der ersten Verbindungsstrebensäule die gleiche Längsachse in einer zweidimensionalen offenen Schnittansicht aufweist.

8. Stent nach Anspruch 6, wobei alle ersten Verbindungsstreben in der ersten Verbindungsstrebensäule parallele Längsachsen in einer zweidimensionalen offenen Schnittansicht aufweisen.

9. Stent nach Anspruch 1, wobei mindestens ein Abschnitt der ersten Verbindungsstreben der ersten Verbindungsstrebensäule asymmetrische geometrische Konfigurationen aufweisen.

10. Stent nach Anspruch 1, wobei ein Anschlussende des proximalen Abschnitts (104) jeder ersten Verbindungsstrebe in der ersten Verbindungssäule (92) mit einer Ausdehnungsstrebe in der ersten Ausdehnungssäule verbunden ist, und ein Anschlussende des distalen Abschnitts (106) jeder ersten Verbindungsstrebe mit einer Ausdehnungsstrebe in der zweiten Ausdehnungssäule verbunden ist.

11. Stent nach Anspruch 1, wobei der proximale Abschnitt (104) jeder ersten Verbindungsstrebe eine Fläche aufweist, die mit mindestens einer Fläche einer Ausdehnungsstrebe in der ersten Ausdehnungssäule verbunden ist, und der distale Abschnitt (106) jeder ersten Verbindungsstrebe mindestens eine Fläche aufweist, die mit einer Ausdehnungsstrebe in der zweiten Ausdehnungssäule verbunden ist.

12. Stent nach Anspruch 1, wobei jede erste Verbindungsstrebe der ersten Verbindungssäule (92) kontralateral mit der ersten und der zweiten Ausdehnungssäule (48, 49) verbunden ist.

13. Stent nach Anspruch 1, wobei mindestens ein Abschnitt des krummlinigen proximalen Abschnitts (104) jeder ersten Verbindungsstrebe der ersten Verbindungsstrebensäule (92) in dichter Nähe zu einem Ausdehnungsstrebenpaar der ersten Ausdehnungssäule (48) liegt.

14. Stent nach Anspruch 1, wobei mindestens ein Abschnitt des krummlinigen proximalen Abschnitts (104) jeder ersten Verbindungsstrebe der ersten Verbindungsstrebensäule (52) parallel zu einem Abschnitt eines Ausdehnungsstrebenpaars der ersten Ausdehnungssäule (48) ist.

15. Stent nach Anspruch 14, wobei mindestens ein Abschnitt des krummlinigen distalen Abschnitts (106) jeder ersten Verbindungsstrebe der ersten Verbindungsstrebensäule (92) in dichter Nähe zu einem Ausdehnungsstrebenpaar der zweiten Ausdehnungssäule (49) liegt.

16. Stent nach Anspruch 1, wobei mindestens ein Abschnitt des krummlinigen distalen Abschnitts (106) jeder ersten Verbindungsstrebe der ersten Verbindungsstrebensäule (92) parallel zu einem Abschnitt eines Ausdehnungsstrebenpaars der zweiten Ausdehnungssäule (49) ist.

## Revendications

1. Extenseur comprenant :
• une première colonne d'expansion (48) comprenant des entretoises d'expansion individuelles formant une multiplicité de paires d'entretoises d'expansion (52, 54) de forme annulaire, deux paires d'entretoises d'expansion adjacentes partageant une entretoise commune, une entretoise d'expansion (52) d'une paire d'entretoises d'expansion de la première colonne d'expansion (48) présentant un segment en marche d'escalier à une section proximale et l'autre entretoise d'expansion (54) de la paire d'entretoises d'expansion présentant un segment en marche d'escalier à une section distale ;
• une seconde colonne d'expansion (49) comprenant des entretoises d'expansion individuelles (52, 54) formant une multiplicité de paires d'entretoises d'expansion (52, 54) de forme annulaire, deux paires d'entretoises d'expansion adjacentes partageant une entretoise commune, une entretoise d'expansion (54) d'une paire d'entretoises d'expansion de la seconde colonne d'expansion (49) présentant un segment en marche d'escalier à une section proximale et l'autre entretoise d'expansion (52) de la paire d'entretoises d'expansion présentant un segment en marche d'escalier à une section distale, une première colonne d'entretoises de connexion (92) comprenant une multiplicité de premières entretoises de connexion individuelles qui relient les première et seconde colonnes d'expansion, chaque première entretoise de connexion dans la première colonne d'entretoises de connexion présentant une configuration géométrique en marche d'escalier,
**caractérisé en ce que** chaque première entretoise de connexion dans la première colonne d'entretoises de connexion présente une section intermédiaire (108) reliée à une section proximale curviligne (104) et une section distale curviligne (106), les sections proximale et distale (104, 106) de chaque première entretoise de connexion de la première colonne d'entretoises de connexion présentant la même longueur, la section intermédiaire (108) présentant un axe longitudinal (124) s'étendant dans une direction longitudinale de l'extenseur et la section proximale (104) de chaque première entretoise de connexion dans la première colonne d'entretoises de connexion (92) étant conjointe de manière controlatérale à une paire d'entretoises d'expansion de la première colonne d'expansion (48) à une section de marche descendante ou de marche ascendante et la section distale lui correspondant (108) étant conjointe de manière controlatérale à une paire d'entretoises d'expansion de la seconde colonne d'expansion (49) à une section de marche descendante ou de marche ascendante.

2. Extenseur selon la revendication 1, chaque première entretoise de connexion dans la première colonne d'entretoises de connexion (92) présentant une extrémité proximale qui s'étend dans une première direction et une extrémité distale qui s'étend dans une seconde direction opposée.

3. Extenseur selon la revendication 1, chaque première entretoise de connexion dans la première colonne d'entretoises de connexion présentant quatre points de pivot (112,114, 116, 118).

4. Extenseur selon la revendication 3, chaque point de pivot (112, 114, 116, 118) présentant au moins un rayon de courbure.

5. Extenseur selon la revendication 1, chaque première entretoise de connexion dans la première colonne d'entretoises de connexion (92) présentant un axe longitudinal (112) qui n'est pas parallèle à un axe longitudinal de l'extenseur.

6. Extenseur selon la revendication 1, chaque section intermédiaire (108) de chaque première entretoise de connexion de la première colonne d'entretoises de connexion (92) présentant un axe longitudinal (124) qui n'est pas parallèle à l'axe longitudinal (120, 122) de la première entretoise de connexion.

7. Extenseur selon la revendication 6, chaque première entretoise de connexion dans la première colonne d'entretoises de connexion présentant le même axe longitudinal dans une vue ouverte en deux dimensions.

8. Extenseur selon la revendication 6, toutes les premières entretoises de connexion dans la première colonne d'entretoises de connexion présentant des axes longitudinaux parallèles dans une vue ouverte en deux dimensions.

9. Extenseur selon la revendication 1, au moins une partie des premières entretoises de connexion de la première colonne d'entretoises de connexion présentant des configurations géométriques asymétriques.

10. Extenseur selon la revendication 1, une extrémité terminale de la section proximale (104) de chaque première entretoise de connexion dans la première colonne de connexion (92) étant conjointe à une entretoise d'expansion dans la première colonne d'expansion, et une extrémité terminale de la section distale (106) de chaque première entretoise de connexion étant conjointe à une entretoise d'expansion dans la seconde colonne d'expansion.

11. Extenseur selon la revendication 1, la section proximale (104) de chaque première entretoise de connexion présentant une surface qui est conjointe à au moins une surface d'une entretoise d'expansion dans la première colonne d'expansion, et la section distale (106) de chaque première entretoise de connexion présentant au moins une surface qui est conjointe à une entretoise d'expansion dans la seconde colonne d'expansion.

12. Extenseur selon la revendication 1, chaque première entretoise de connexion de la première colonne de connexion (92) étant conjointe de manière controlatérale aux première et seconde colonnes d'expansion (48, 49).

13. Extenseur selon la revendication 1, au moins une partie de la section proximale curviligne (104) de chaque première entretoise de connexion de la première colonne d'entretoises de connexion (92) étant à proximité rapprochée d'une paire d'entretoises d'expansion de la première colonne d'expansion (48).

14. Extenseur selon la revendication 1, au moins une partie de la section proximale curviligne (104) de chaque première entretoise de connexion de la première colonne d'entretoises de connexion (52) étant parallèle à une partie d'une paire d'entretoises d'expansion de la première colonne d'expansion (48).

15. Extenseur selon la revendication 14, au moins une partie de la section distale curviligne (106) de chaque première entretoise de connexion de la première colonne d'entretoises de connexion (92) étant à proximité rapprochée d'une paire d'entretoises d'expansion de la seconde colonne d'expansion (49).

16. Extenseur selon la revendication 1, au moins une partie de la section distale curviligne (106) de chaque première entretoise de connexion de la première colonne d'entretoises de connexion (92) étant parallèle à une partie d'une paire d'entretoises d'expansion de la seconde colonne d'expansion (49).
